# EUROPEAN PATENT SPECIFICATION

(11) **EP 2 195 048 B1**
(45) Date of publication and mention of the grant of the patent: **12.12.2018**
(21) Application number: 08836880.8
(22) Date of filing: 10.10.2008
(51) Int. Cl.: A61M 5/142

(54) **CARRIER FOR AN INFUSION SYSTEM**
TRÄGER FÜR EIN INFUSIONSSYSTEM
SUPPORT POUR UN SYSTÈME DE PERFUSION

(30) Priority: 11.10.2007 US 979272 P; 11.10.2007 US 979279 P
(43) Date of publication of application: 16.06.2010
(73) Proprietor: Roche Diabetes Care GmbH, 68305 Mannheim (DE); F. Hoffmann-La Roche AG, 4070 Basel (CH)
(72) Inventor: KAUFMANN, Heiner, CH-3008 Bern (CH); STUDER, Gérald, CH-2545 Selzach (CH)
(74) Representative: Rentsch Partner AG
(86) International application number: PCT/EP2008/008573
(87) International publication number: WO 2009/046989

(56) References cited:
- EP-A- 1 338 295
- EP-A- 1 754 890
- WO-A-96/37244
- WO-A-2007/035666
- WO-A-2007/065944
- WO-A-2007/071255
- FR-A- 2 734 162
- US-A1- 2001 056 262
- US-A1- 2003 106 917
- US-B1- 6 659 978

## Description

### RELATED APPLICATION

This application claims priority to and benefit of US Provisional Patent Application Ser. No: 60/979,272 and US Provisional Patent Application Ser. No: 60/979,279, both filed on October 11, 2007.

### TECHNICAL FIELD

The present invention generally relates to systems for attaching a medical device to human body. Specifically it relates to a new carrier system for an infusion system with improved wearing comfort and handling.

### BACKGROUND

Medical devices that pump medication into an individual is known and commonly used in the medical industry. Typically the medication that is delivered from such medical devices depends on the medical condition that is sought to be treated. For example, it is getting increasingly common to deliver insulin using an insulin pump to treat a diabetic patient.

Typically, the medical pump devices use a reservoir or a cartridge that contains the medicine to be delivered. Due to miniaturization of products there is an expectation that such infusion systems that carry the medication would be smaller thereby making the ability to wear and operate the system more discreet. Since different users typically like to hide the infusion pump in different places, it is important to provide an interface that will allow a user this flexibility. As the size of the infusion system becomes smaller, there is a tendency to attach the infusion system directly to the skin of the user. In most cases, the medication is infused by adhering the infusion system in the stomach area. However, the human skin is very flexible and pliable and typically in the stomach area often exists skin folds. The application of conventional carrier systems between such skin folds is very uncomfortable. Furthermore, the skin tends to stretch-out more in the vertical axis than in the horizontal axis. Large pressure sensitive adhesives in the vertical axis inhibits skin movement and introduces stress. Furthermore, when such devices are worn on the skin, perspiration (skin breathing) is reduced and this leads to more skin irritation. In addition in conventional attachment systems, the pressure sensitive adhesives are not well accessible.

Therefore there is a need in the industry to design a carrier where the infusion system can be properly carried by the user, also does not hinder in the proper Operation of the infusion device and is comfortable for carrying on the skin of the user.

Carriers attachable to the body of a user for holding an infusion pump device are known from the state of the art. WO 2007/065944 A1 discloses such a carrier, with a carrier frame, a first guiding means on the carrier frame, a second guiding means on the infusion pump. When the infusion pump is attached to the carrier frame, the first and the second guiding means mate with each other to hold the infusion pump in place. The carrier frame comprises an adhesive layer for attaching the carrier to the skin of a user. Similar infusion pump devices with adhesively attachable carriers are also disclosed in EP 1754890 A2 and WO 2007/071255 A1.

WO 96/37244 A1 discloses a medical device to be attached to the body of a user, in which the device is attached to the adhesive layer only in a central area. A similar device is shown in US 2001/0056262 A1, where the device is connected to the adhesive layer on three points arranged in a triangle. FR 2734162 relates to a reusable controller which can be coupled to a disposable drug-containing unit. WO 2007/035666 relates to electrokinetic pumps for medical applications. US 2003/106917 relates to a holster with a clip portion for a user's belt and a harness portion for holding a device such as an infusion pump. US 6659978 relates to a tank unit, pump unit, and controller attached to a wrist strap so that they can be easily connected and disconnected from each other.

### SUMMARY

It is against the above background that the present invention proves certain unobvious advantages and advancements over the prior art.

The present invention generally relates to an interface for carrying an infusion system on a user such that the infusion system can be concealed from view. In the present invention a system for attaching a medical device to human skin is generally disclosed. The invention discloses a new carrier system for a drug delivery system with improved wearing comfort and handling. It is generally difficult to successfully adhere a rigid device to the skin for a prolonged period of time due to the rigidity of the device, the heightened center of gravity above the adhesion level, and the flexibility and pliability of the skin. Anything attached to the skin applies stress and shear forces to the skin resulting in low wearing comfort and enhancing the detachment of the system. Therefore the aim of the present invention is to minimize the negative influence of a carrier system to the skin in order to reduce the chance of detachment from the skin. Thus the present invention maximizes the wearing comfort and facilitates the handling during application, use and removal of the carrier system.

These and other features and advantages of the present invention will be more fully understood from the following detailed description of the invention taken together with the accompanying claims. It is noted that the scope of the claims is definitely by the recitations therein and not by the specific discussion of the features and advantages set forth in the present description.

### BRIEF DESCRIPTION OF THE DRAWINGS AND DETAILED DESCRIPTION

The following detailed description of the embodiments of the present invention can be best understood when read in conjunction with the following drawings, where like structure is indicated with like reference numerals and in which:

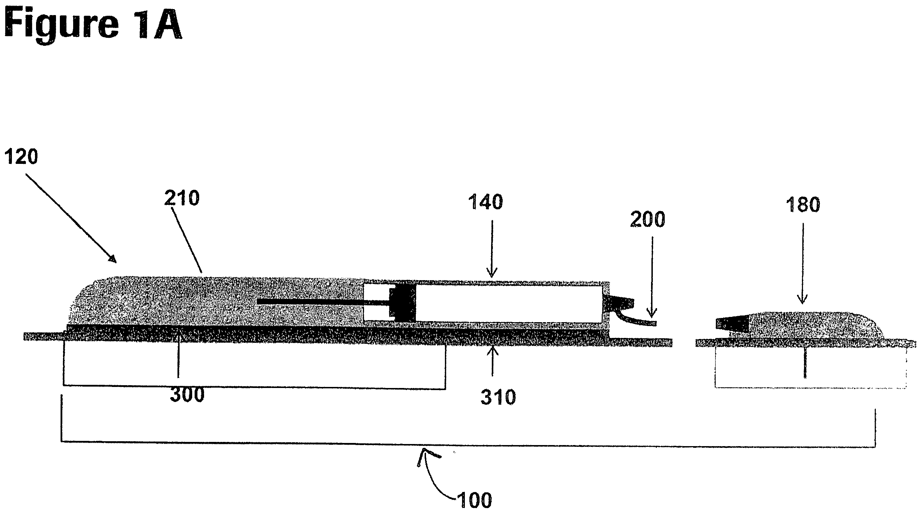

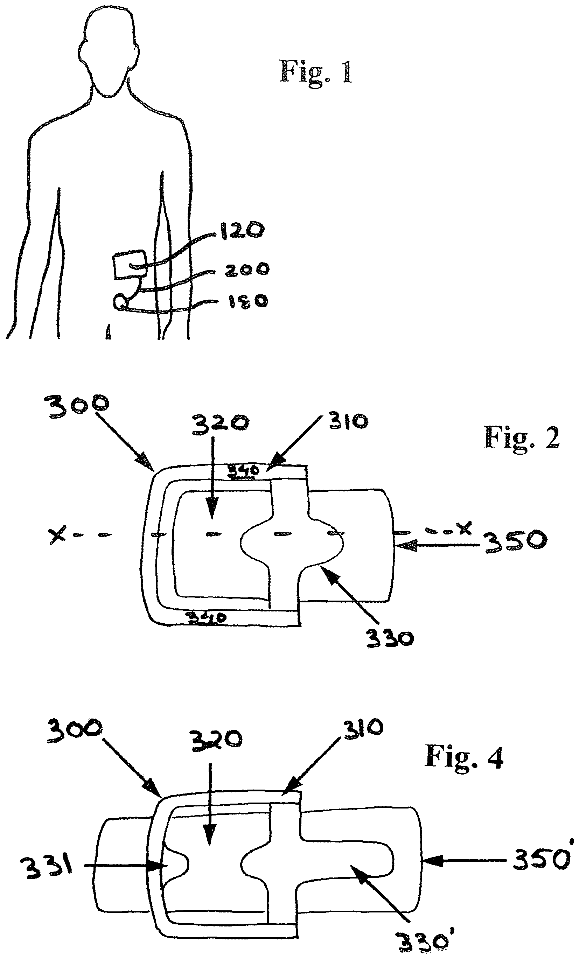

**Fig. 1A** is a perspective view of the infusion delivery system in accordance with the teachings of the present invention;
**Fig. 1** is a frontal view of a human body with attached drug delivery device in horizontal axis;
**Fig. 2** is a top plan view of a carrier with small pressure sensitive adhesive;
**Fig. 3** is a top plan view of an attached infusion delivery system. The carrier has a small pressure sensitive adhesive and is not visible;
**Fig. 4** is a top plan view of a carrier with large pressure sensitive adhesive;
**Fig. 5** is a top plan view of an attached drug delivery system. The carrier has a large pressure sensitive adhesive visible on both sides of the delivery system;
**Fig. 6** is a top plan view of a carrier frame with two cut-offs;
**Fig. 7** is a top plan view of a carrier frame with four cut-offs;
**Fig. 8** is a top plan view of a carrier with four cut-offs and connection bars to the edges;
**Fig. 9** is a top plan view of a carrier for attaching the drug delivery system in a vertical position;
**Fig. 10** is a top plan view of an attached drug delivery system in a vertical position;
**Fig. 11** is a top plan view of a carrier for attaching the drug delivery system in a vertical position;
**Fig. 12** is a top plan view of an attached drug delivery system in a vertical position;
**Fig. 13** is a top plan view of a carrier frame with one transversal connection bar and two cut-offs;
**Fig. 14** is a top plan view of a carrier with double fixation and one cutoff;
**Fig. 15** is a perspective view of a drug delivery device with concave linear;
**Fig. 16** is a perspective view of a drug delivery device with concave linear
**Fig. 17** is a perspective view of a drug delivery device with convex linear;
**Fig. 18** is a perspective view of a drug delivery device with convex linear;
**Fig. 19** is a top view of a carrier with a cross bar frame and a fastening means;
**Fig. 19A** is a top view of a carrier with a cross bar frame and a fastening means wherein the adhesive layer is shaped like a peanut;
**Fig. 20** is a top plan view of the attached infusion pump to the carrier of Fig. 19;
**Fig. 21** is a side view of a drug delivery device with a snap fastener;
**Fig. 22** is a side view of a carrier frame with a snap fastener;
**Fig. 23** is a side view of a carrier frame with a snapped drug delivery device;
**Fig. 24** is a top plan view of a carrier with two snap fasteners in the middle;
**Fig. 25** is a top plan view of a carrier with two snap fasteners at the end;
**Fig. 26** is a top plan view of a drug delivery device with a snap fastener;
**Fig. 27** is a top plan view of a drug delivery device with two snap fasteners;
**Fig. 28** is a top plan view of a carrier with infusion needle;
Fig. 29 is a top plan view of a carrier of Fig 19 with an integrated needle;
Fig. 30 is a side view of a carrier with linear guiding and infusion needle;
Fig. 31 is a top view of a carrier with a connector;
Fig. 32 is a top view of a carrier with a hook on connector;
Fig. 33 is a perspective view of the carrier with an attached wrist band;
Fig. 33A is a perspective view of the infusion pump as attached to the carrier of Fig. 33;
Fig. 34 is a perspective view of the carrier attached to the infusion pump as a side clip on;
Figs. 35-38 are top views of the carrier attached to the infusion pump as a top clip on.
Fig. 39 is a perspective view of a carrier with hooks; and
Fig. 40 is a perspective view of the infusion pump as attached to the carrier of Fig. 39.

### Detailed Description

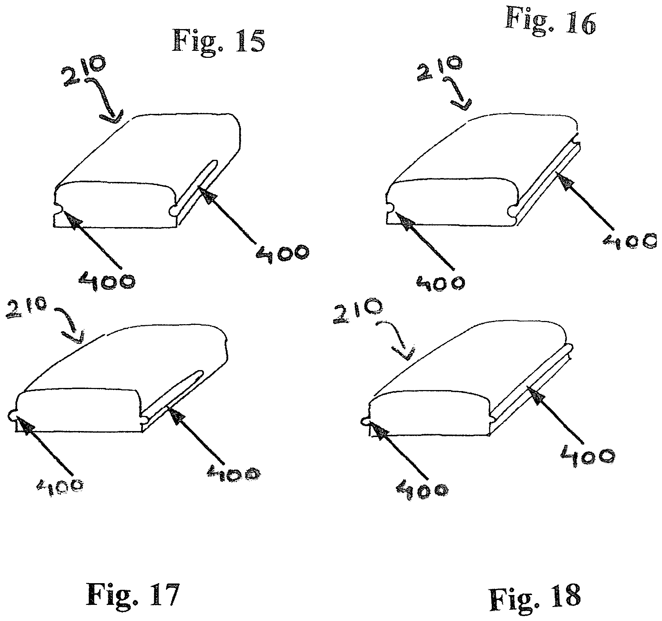

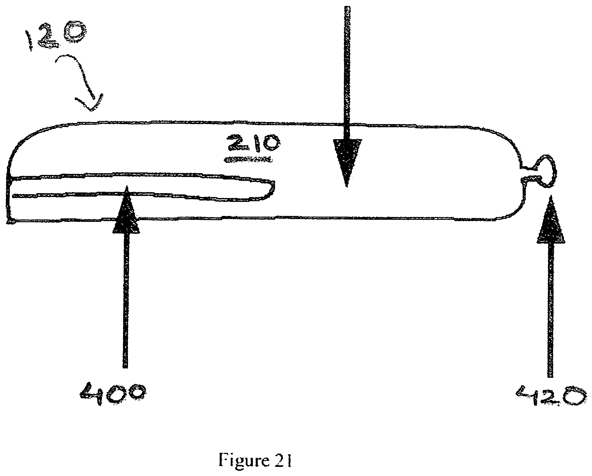

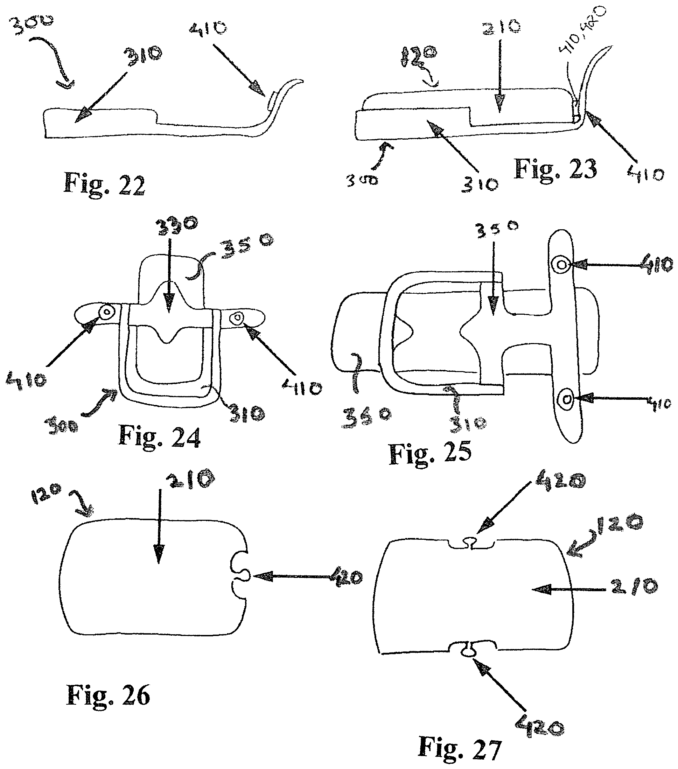

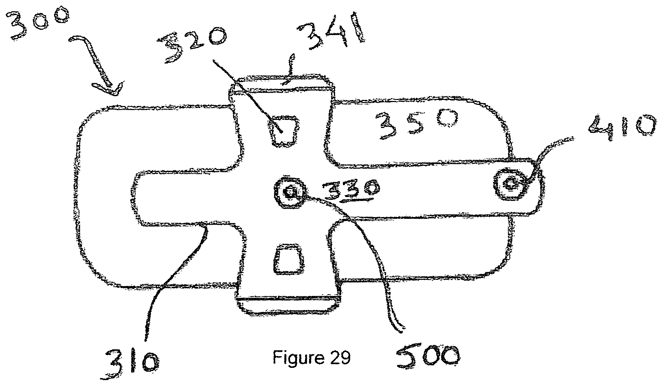

The present invention describes a new skin attachment system for a drug delivery device with improved wearing comfort and handling. The attachment system, a so-called carrier, is adapted to be disposed between a drug delivery device and human skin for reliably attaching the device to the skin for an extended period of time. Figs. 15-40 represent background art that is useful for understanding the invention.

Referring in particular to Figs. 1A and 1, an infusion delivery system is generally represented by reference numeral 100. As can be seen in the drawing, the infusion delivery system comprises an infusion pump 120, a fluid storing means 140, an infusion set (not completely shown) 180 that is connected to the fluid storing means through a tube 200. The infusion pump shown in the drawings is an insulin pump 120 where the medication in the fluid storing means 140 is insulin. Although an insulin pump is shown, it must be understood that this invention is not limited to insulin pumps but to any pump that can be used to deliver medication.

Although not expressly shown in the figures, the infusion pump 120 has at least one control (to control the infusion of medication from fluid storing means 140. Alternatively, the infusion pump 120 may have display and/or controls or be void of both display and/or controls. Although not shown in the drawings the infusion pump 120 may be controlled remotely to dispense medication using a remote control device such as a smart phone, a PDA or any other devices including but not limited to mobile devices. Although not specifically mentioned, the infusion pump 120 may be a one time use pump such that after the dispensing of the medication the pump is disposed.

With continued reference to Fig. 1A, the infusion pump 120 comprises a housing 210 that houses a drive unit for dispensing the medication from the fluid storing means 140 to the user through the infusion set 180. The infusion system 100, further comprises a carrier 300 that is adapted to act as an attachment means for the infusion pump 120 to the body. It must be understood although the carrier 300 as shown is suitable for attaching a body mounted infusion pump, it must be understood that the carrier can be adapted to hold a small infusion pump where the carrier is not attached to the skin.

As shown, the housing 210 is removably attached to a carrier 300 on one side of the carrier 300. On the other side of the carrier, the carrier 300 is removably attached to the skin of the user via suitable adhesion means such as a plaster. In use, the user attaches the carrier 300 with the adhesive layer onto the skin and then attaches the pump 120 to the carrier. Alternatively, the user may attach the infusion pump to the carrier and then attach the carrier to the skin. Fig. 1 shows an infusion pump 120 that is attached to the body of a user using the carrier 300 (not shown) of the present invention. Although a particular position i.e the abdomen of the user is shown in the drawing, it must be understood that the infusion pump is capable of being worn anywhere in the body including the back.

With particular reference to Figs. 2 to 14, a carrier having different configuration is generally shown and represented by reference number 300. As shown in this configuration, the carrier 300 consists of a frame structure 310. The frame structure can be manufactured using a rigid material such as plastic or a flexible and soft material such as textile. As seen in the drawings, the frame 310 is provided with cut off areas 320. Depending on the frame structure 310 the resulting carrier 300 may have one or several cut off areas 320.

With continued reference to Figs. 2 to 14, the carrier 300 comprises an adhesive layer 350 that is on a side opposite to the side where the infusion pump 120 is attached to the carrier 300. In particular, the adhesive layer 350 is a pressure sensitive adhesive such that upon application of pressure the carrier attaches to the skin of the user as shown in Fig. 1. Pressure sensitive adhesives consist generally of silicone, butyl or acrylic components that are formulated in such a way so that they adhere to the skin. For example, as can be seen in Fig 2, the adhesive layer 350 doesn't extend to the edges of the device in the vertical axis. This allows the skin to be naturally stretched-out in the vertical axis during body movements. Thus less stress is introduced resulting in good wearing comfort.

In order to attach the frame structure 310 to the adhesive layer 350, the carrier 300 is provided with an area of attachment generally represented by reference numeral 330. The area of attachment 320 of the adhesive layer 350 to the frame 310 is a central spot or a line along the horizontal axis X-X (as shown in Fig. 2). This configuration allows the carrier 300 to rotate freely around the horizontal axis, thereby providing an increased flexibility in the use of the infusion pump 120.

In order to attach the infusion pump 120 to the carrier 300, the frame structure 310 is provided with a guiding means 340 that is part of the frame structure 310. In one embodiment, the guiding means 340 are ramps that mate with the infusion pump housing 120 to hold the pump in the carrier 300. In other embodiments, the guiding means 340 could be indents where the pump slides into the carrier frame 300.

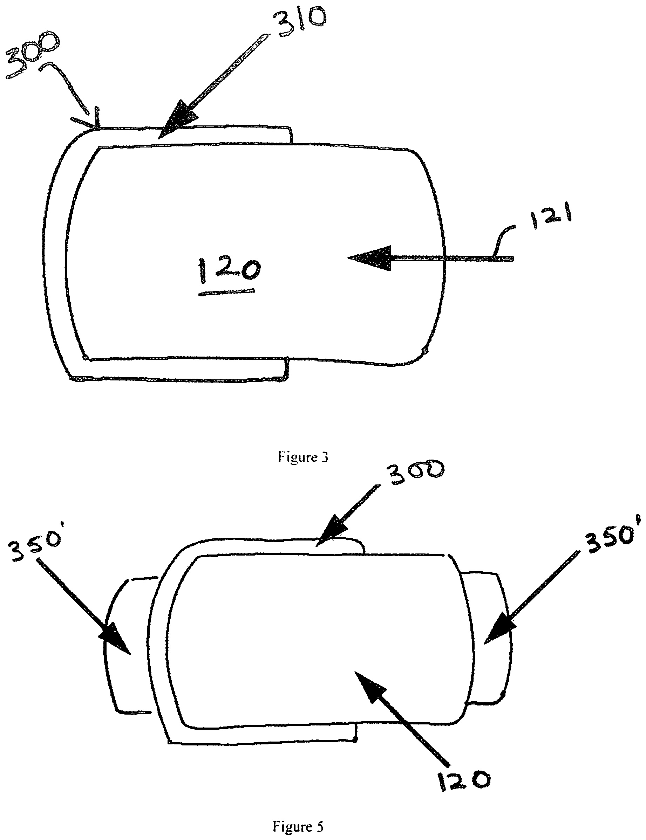

With particular reference to Fig. 3, an infusion pump 120 as attached to the carrier 300 is shown. As can be seen in this embodiment, the adhesive layer 350 is not visible once the infusion pump 120 is attached to the carrier 300. In this embodiment, the infusion pump 120 is pushed against the carrier 300 in the direction as shown by arrow 121.

As mentioned above, since different frame structures result in different shapes of the carrier 300, different designs are shown in Figures 4 to 14. With particular reference to Figs. 4 and 5, a carrier 300 is shown like in Fig 2 and 3. The carrier is identical to the carrier of Fig. 2 and 3 except that the adhesive layer 350' is larger. In addition, the attachment point 330' is also larger compared to the embodiments of Fig 2 and 3. In addition, the carrier 300 is also provided with an additional attachment area 331 to attach the adhesive layer 350' to the frame 310. As seen in Fig 5, when the infusion pump 120 is attached to the carrier 300, the adhesive layer 350' is visible to the user. Such an embodiment may have advantages since the corner of the adhesive surface can be provided with a gripping member such that it allows for easy removal of the carrier 300 from the body of the user.

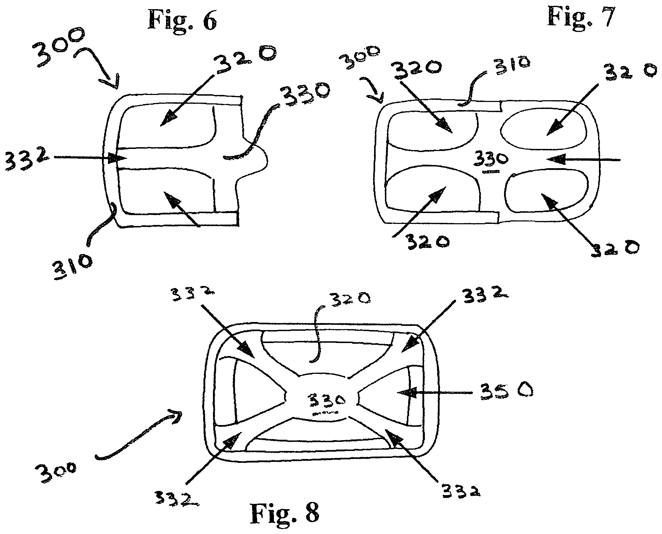

As shown in Figs 6, 7 and 8 another design for the carrier 300 having a frame 310 is shown. As can be seen in Fig 6, the attachment area 330 is connected to the frame 310 by a connection bar 332. The connection bar may be made of the same material as the frame 310. In Figs 7 and 8, the frame 310 design results in 4 cut off areas 320 and a central point of attachment 330.

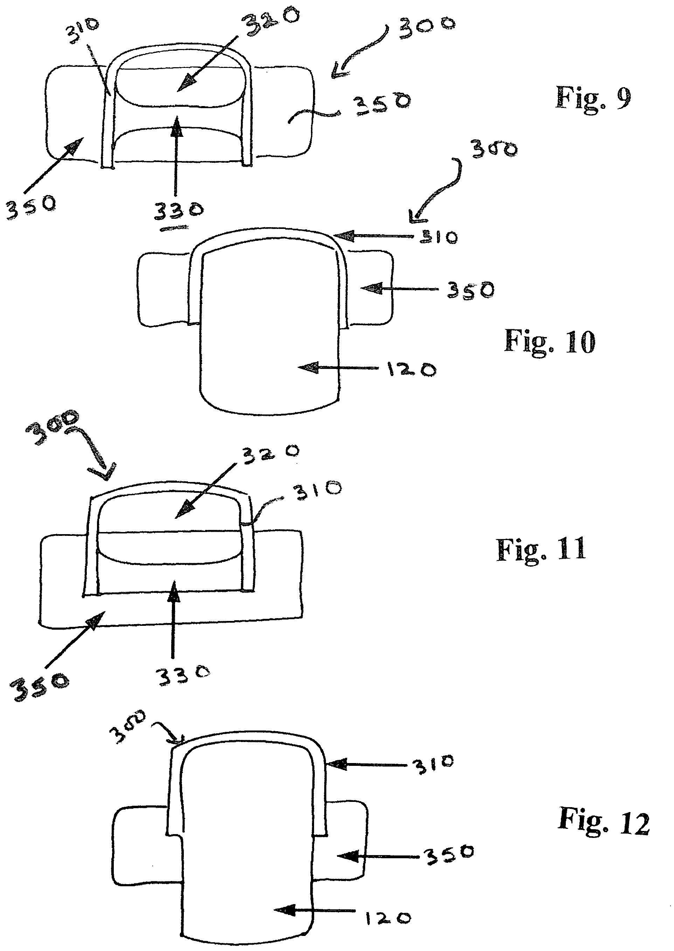

With particular reference to Figs. 9, 10, 11 and 12, represents a carrier 300 capable of being attached to the skin of the person in a vertical position. As mentioned before since the carrier frame 310 is attached to the adhesive layer 350 at a central attachment point it can be oriented in a manner suitable for the user.

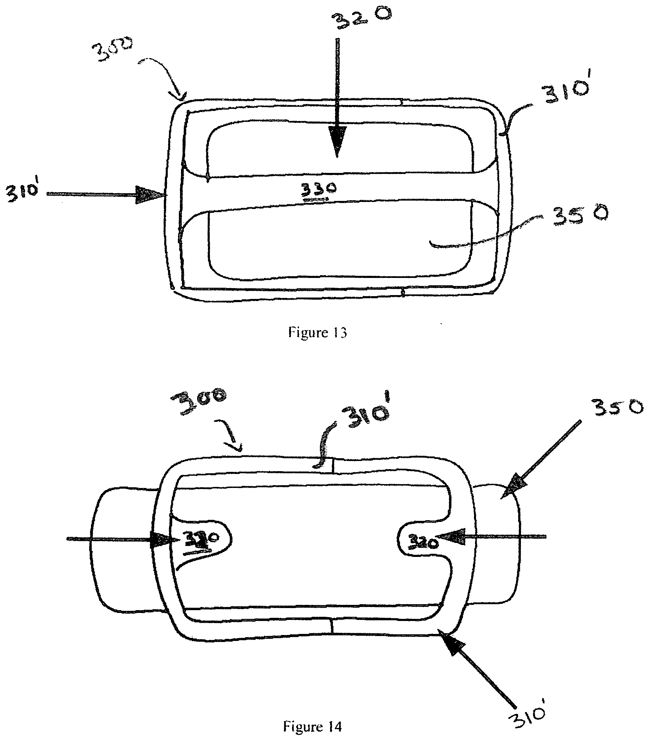

Referring in particular to Figs. 13 and 14, a carrier 300 has a frame structure 310' that has the same size as the infusion pump housing.

Contrary to carrier structure with no cut-offs, a carrier with a frame structure and cut-offs has several advantages. First of all there exists a better accessibility of the pressure sensitive adhesive. The pressure sensitive adhesive can be pressed on by fingers after application and can easily visually controlled by the patient. In addition the pressure sensitive adhesive is better accessible for removal and therefore causes less pain. Furthermore the skin respiration of the skin portion which is covered by the pressure sensitive adhesive is better, because there is always space between the base surface of the device and the pressure sensitive adhesive. The enhanced air circulation inhibits skin irritation. Aside from that the frame structure of the carrier makes the carrier more elastic and flexible. A flexible structure is a key component for the wearing comfort, because human skin is very flexible, too.

As mentioned above, the infusion pump 120 is attached to the carrier 300 by the guiding means 340 of the carrier. In order for the infusion pump to mate with the guiding means, the infusion pump is provided with complementary guiding means.

Examples in fig. 15-40 are not embodiments according to the invention, but represent background art which is useful for understanding the invention.

With particular reference to Figs. 15 to 18, an infusion pump housing 210 is provided with a liner guiding means 400. As shown in Figures, the linear guiding means 400 can be concave or convex and can extend the entire length of the infusion pump housing or can partially extend through the length of the infusion pump housing.

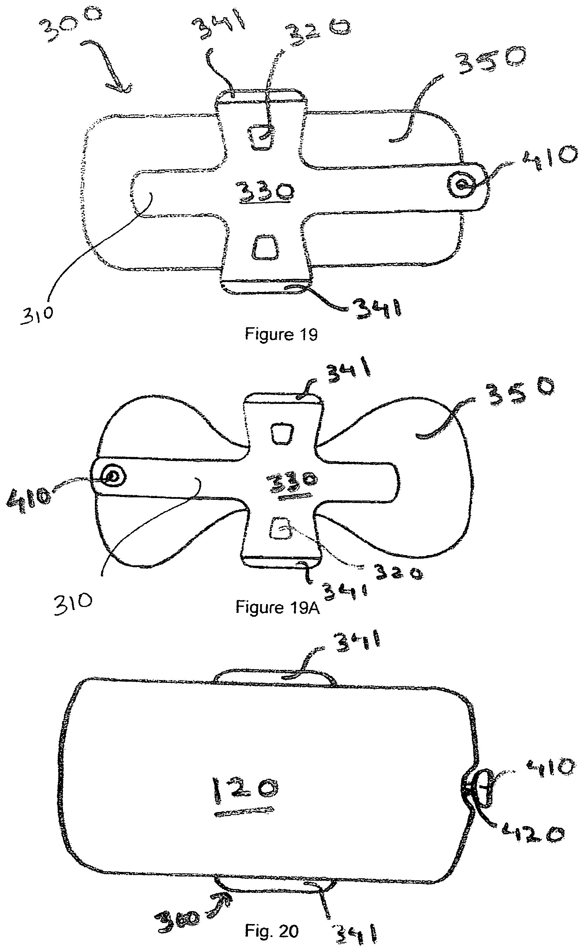

With particular reference to Figs. 19 and 19A, the carrier 300 is provided with a relatively small frame 310 with an attachment point 330. As can be seen from the figure, this results in a cross design of the carrier 300. The carrier has 2 small cut off areas 320 and the area of the attachment 330 of the adhesive layer 350 to the carrier frame 310 is a central line along the horizontal axis. In addition, carrier 300 is also provided with a complementary ramp or guide 341 to mate with the linear guide means in the infusion pump. As shown in Fig 20, once the infusion pump 120 is attached to the carrier 300, it is very small, discreet and easy to move along the horizontal axis.

With reference to Figs. 19, 22 to 25, shows a design of the carrier 300, wherein the carrier is provided with at least one fastening means 410. The fastening means 410 can be any traditional fastening means such as a snap fastener similar to textile snap fastener. In order to securely attach the fastening means 410 with the infusion pump 120, the infusion pump 120 is provided means 420 (as shown in Figs. 20, 21, 23, 26 and 27) that can mate with the fastening means 410 on the carrier 300.

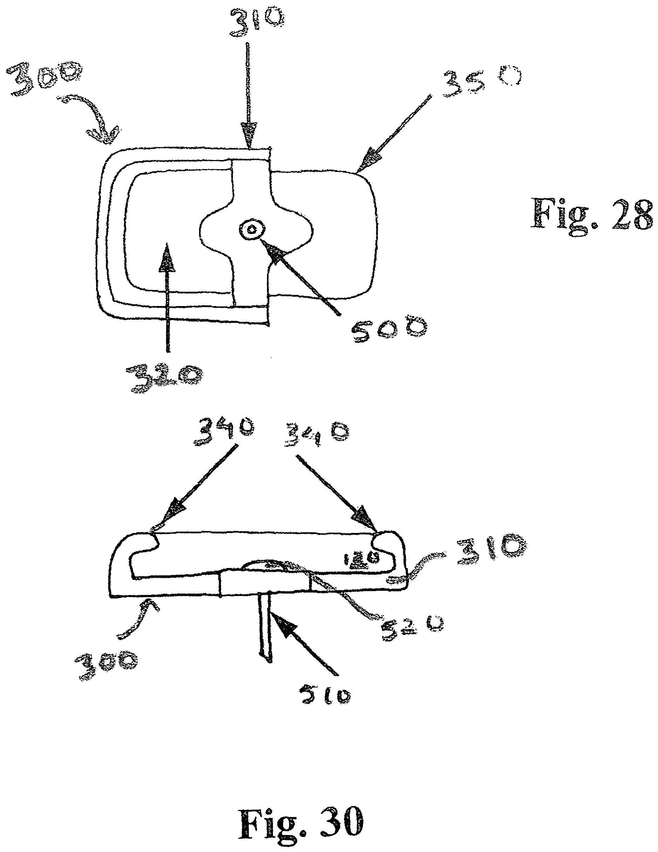

With particular reference to Figs. 28 to 30, the carrier 300 may also be provided with an integrated needle 500. As shown in the drawings the needle 500 is positioned in the center carrier 300 serving for delivery of a fluid into the body. The needle 500 consists of a cannula 510 and a septum 520. The cannula 510 is inserted into the tissue. The septum 520 is formed in a carrier 300 in order to seal off the cannula hermetically from the outside. Although not shown in the drawings, a connecting needle is part of the infusion pump 120. This connecting needle is connected to the fluid channel 140 (as shown in Figure 1A). When the infusion pump 120 is connected to the carrier 300 the connecting needle pierces the septum 520 of the carrier 300, so that the 140 is connected fluidically to the cannula. It must be understood that the infusion pump 120 can be separated from the carrier 300 and repeatedly connected to the carrier 300. Each time the connection is established, the connecting needle pierces the septum 520. The septum has the property of hermetically sealing off the cannula form the outside when the connecting needle is withdrawn form the septum upon release of the connection between the carrier and the human body. The cannula itself can be made of steel or a more flexible material. A flexible cannula is introduces into the tissue by means of a puncture needle, in which case, after introduction of the cannula, the puncture needle is removed again and only the flexible cannula remains in the tissue. In case of a flexible cannula a second sealing element, a additional septum, may be required.

As shown in the drawings and described above, a carrier 300 allows attachment of an infusion pump 120 to the human skin for an extended period of time through an adhesive layer 350. The infusion pump can easily be joined and separated from the carrier. This allows the infusion pump to be operated (e.g. to change the insulin reservoir) without detaching the skin-contacting surface of the medication delivery system from the skin.

The construction embodied in the present carrier system allows the skin to move more freely than in the case of prior art systems resulting in a better wearing comfort. Furthermore a fastening system situated at the side of the device allows to design thinner delivery systems.

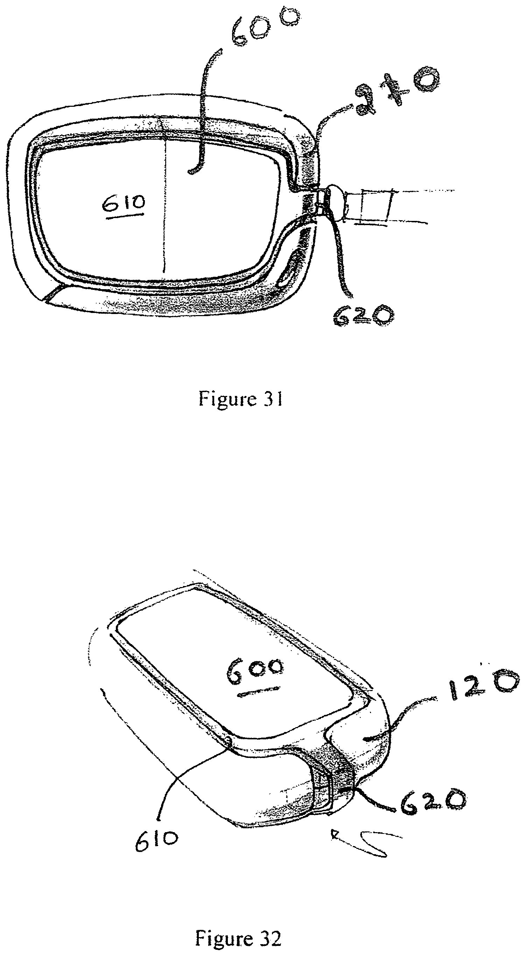

Although a carrier 300 with an adhesive layer 350 is described above, it is possible to design a carrier for carrying infusion pump that does not include an adhesive. When such a carrier is designed, the user does not attach the infusion pump to the skin but is free to carry the infusion pump in the carrier. Referring to Figs. 31 and 32, a carrier 600 without an adhesive layer is generally shown. Although not shown in the drawings, the carrier 600 is provided with means that can help mate it with the infusion pump housing 210. The carrier 600 comprising a housing 610 that is generally made up of a rigid material such as plastic. Alternatively, it may also be made of semi rigid material or a soft material such as a fabric. On one end of the housing 610 is provided with a connector 620 such that it can be attached to a clip or attached to a key ring, or a pouch such that it can hang from the person's neck. In order to reduce the size of the carrier 600, the housing of the carrier may be smaller than the pump housing. In this embodiment, the connecter 610 is such that it attaches to the housing in a hook like manner. In this way the connector can also be provided with means such that the infusion pump may be carried in different ways by the user.

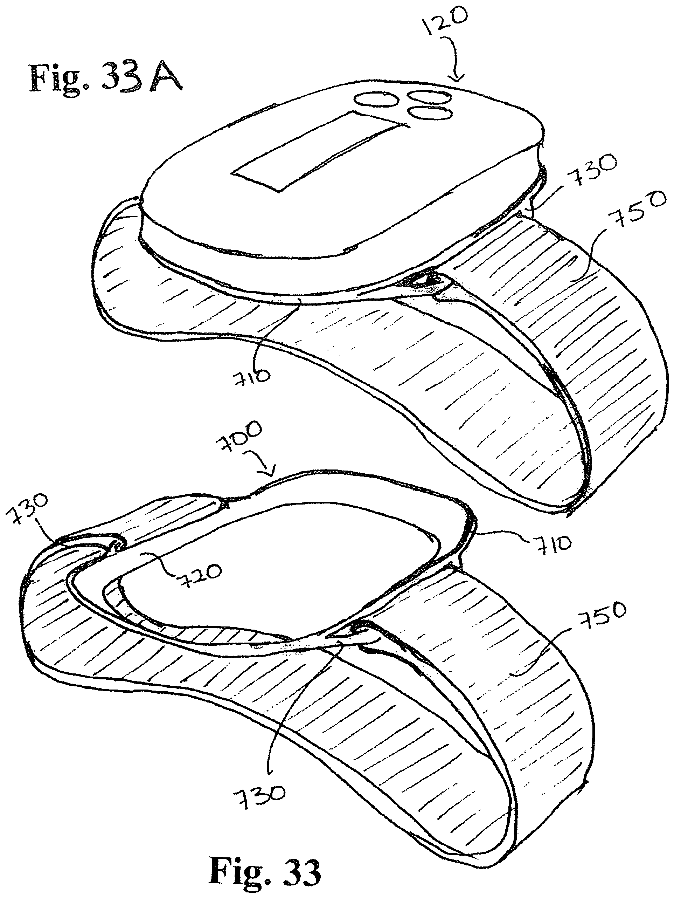

Referring in particular to Fig. 33 and 33A, a carrier 700 that can be attached to a wrist band is generally shown. As can be seen from the drawing, the carrier 700 has a frame 710. The interior of the frame has ramp 720 such that an infusion pump housing snap fits into the frame 710. In opposite ends of the frame 710 are provided connecting means 730 such that a flexible wrist 750 band can be such attached to the carrier 700. As shown in Fig. 33A, once the infusion pump is connected to the carrier, then it can be carried around like a watch. Although a wrist band is shown in the drawings, it must be understood that such a carrier can be used to attach a belt or a waist band or a band that can be tied in the legs.

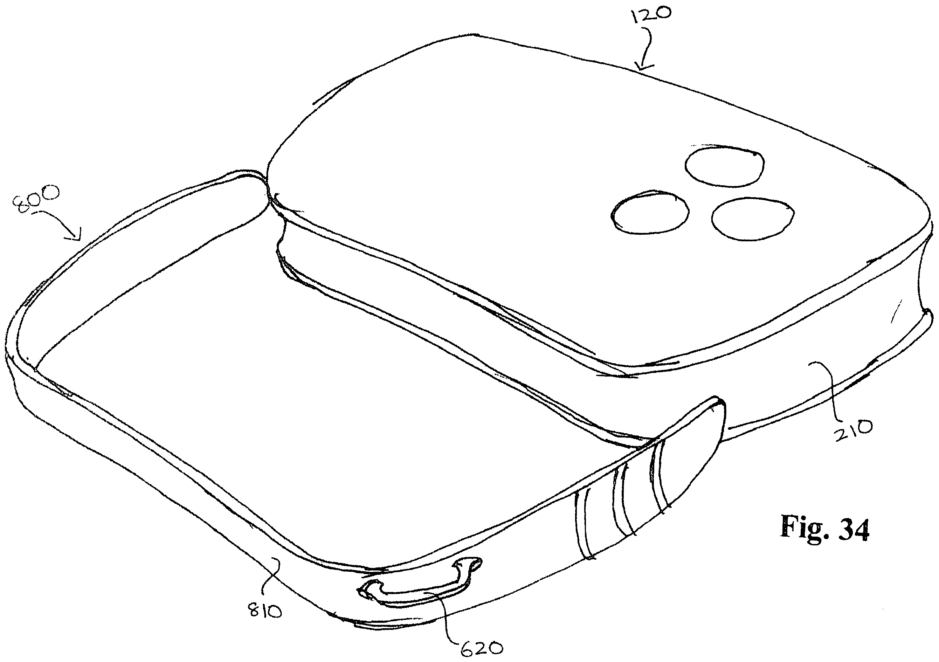

In Fig. 34 the carrier 800 is shown. As can be seen from the drawing, the carrier comprises a frame 810 that can be attached to the infusion pump housing 210 such that it clips around the housing. This structure of the carrier has advantages in that on this carrier frame, a connector 620 of Fig. 31 can be attached.

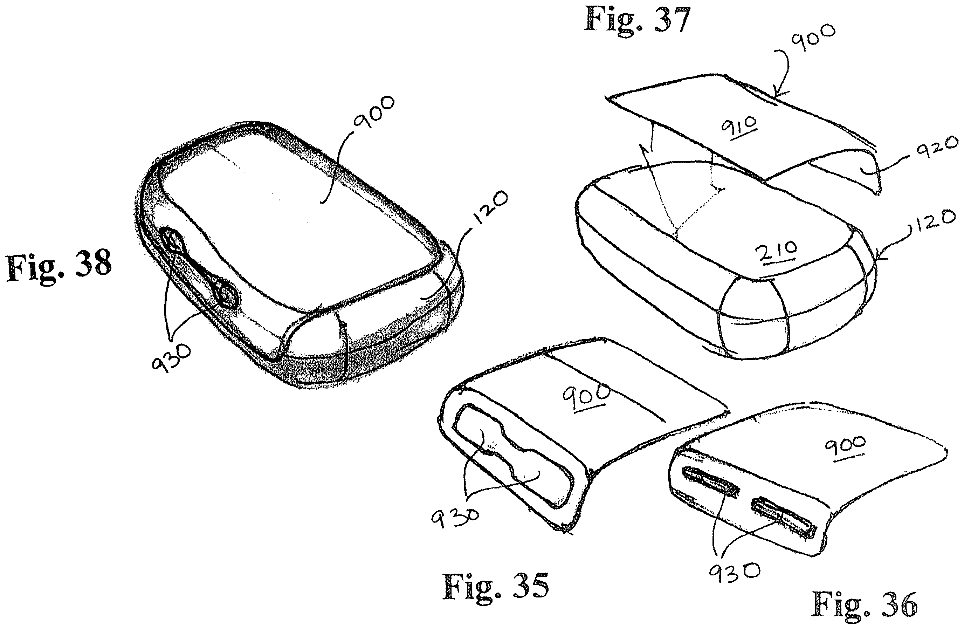

Referring in particular to Figs. 35-38, a clip on carrier is generally shown and represented by reference numeral 900. As can be seen from the drawing, the carrier 900, clips on to the housing 210 of the infusion pump 120. In this design, the carrier has a flat surface 910 and a surface 920 that is substantially perpendicular to the flat surface 910 such that it clips one of the sides of the infusion pump housing. As shown, the carrier 900 also includes cut outs 930 such that operational buttons can be easily accessed by the user.

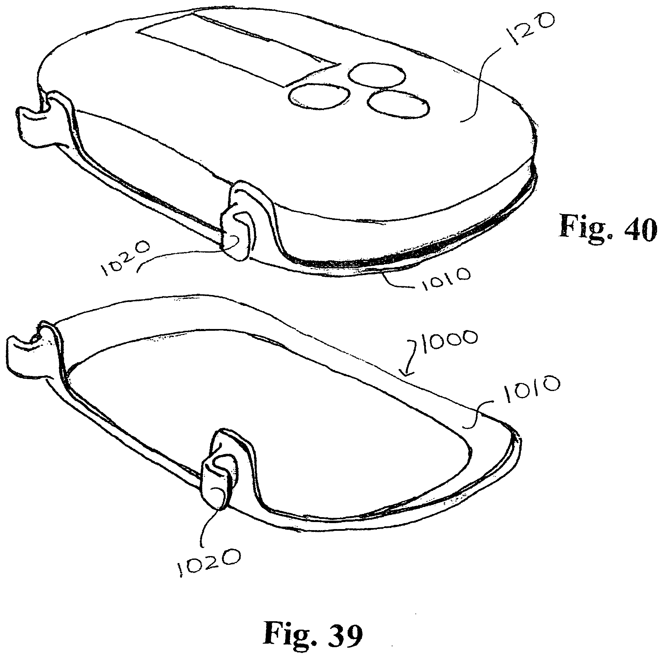

Referring in particular to Figs. 39 and 40, another design of the carrier is generally shown. As can be seen from the drawings, the carrier 1000 is similar in design to the carrier as shown in Fig. 33, except that it does not have a wrist band attached to the carrier. As can be seen in the drawings, the carrier 1000 has a frame 1010 that has the same shape as the infusion pump 120. In addition, the frame 1010 is provided with at least one hook or clip 1020 that is integral with the carrier body. One advantage of this design is the fact that the hook 1020 can be clipped to any part of the clothing such as a belt or a women's undergarment.

Skilled artisans appreciate that elements in the figures are illustrated for simplicity and clarity and have not necessarily been drawn to scale. For example, the dimensions of some of the elements in the figure may be exaggerated relative to other elements to help improve understanding of the embodiment(s) of the present invention.

The description of the preferred embodiment is merely exemplary in nature and is in no way intended to limit the invention or its application or uses.

It is noted that terms like "preferably", "commonly", and "typically" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

For the purposes of describing and defining the present invention it is noted that the term "substantially" is utilized herein to represent the inherent degree of uncertainty that may be attributed to any quantitative comparison, value, measurement, or other representation. The term "substantially" is also utilized herein to represent the degree by which a quantitative representation may vary from a stated reference without resulting in a change in the basic function of the subject matter at issue.

## Claims

1. A carrier (300) for holding an infusion pump (120) such that the infusion pump can be carried on the body of a user, the carrier comprising:
a carrier frame (310, 310');
an adhesive means (350, 350'), such that the carrier can be attached to the skin of a user;
a first guiding means (340, 341) on the carrier frame that can mate with a complementary second guiding means (400) provided on the infusion pump, in order to attach the infusion pump to the carrier and to hold the infusion pump in place on the carrier frame;
**characterized in that**
the adhesive means comprises an attachment area (320), the attachment area being
restricted to either
a central spot or a line along a horizontal axis (X-X) of the adhesive means; and
the carrier frame includes one or more attachment areas (330, 330', 331), where the carrier frame is attached to the attachment area of the adhesive means.

2. The carrier according to claim 1, wherein the carrier frame (310) comprises two attachment areas (330', 331) arranged on a line.

3. The carrier according to any of the preceding claims, wherein the carrier frame (310, 310') includes at least one cut off (320), such that the adhesive means (350) is accessible by a user.

4. The carrier according to any of the preceding claims, wherein the carrier frame (310, 310') further comprises at least one fastening means (410) for attaching (420) an infusion pump (120) to the carrier (300).

5. The carrier according to claim 4, wherein the at least one fastening means (410) comprises a snap fastener.

6. The carrier according to any of the preceding claims, wherein the carrier frame (310, 310') further comprises an integrated needle (500) for delivery of a fluid into a body.

7. The carrier according to any of the preceding claims, wherein the carrier (300) comprises an integrated needle (500) for delivery of a fluid into a body, with a cannula (510) to be inserted into the tissue of the body, e.g. a steel cannula or a flexible cannula, and a septum (520) hermetically sealing off the cannula from the outside, such that when an infusion pump (120) is connected to the carrier, a connecting needle of the infusion pump that is connected to a fluid channel (140) of the infusion pump can penetrate the septum (520) and establish a fluid connection between the fluid channel (140) of the infusion pump (120) and the cannula (510).

8. The carrier according to any of the preceding claims, wherein the carrier frame (310, 310') is connected to the attachment area (330, 330') of the carrier frame by a connection bar (332).

9. An infusion system (100) comprising an infusion pump (120), and a carrier (300) according to any of the preceding claims, wherein the infusion pump comprises a second guiding means (400) that can mate with a first guiding means (340, 341) of a carrier frame (310, 310') of the carrier, and that the infusion pump can be attached to the carrier and hold in place on the carrier frame by mating the first guiding means and the second guiding means.

10. The infusion system of claim 9, wherein the first guiding means (340, 341) of the carrier frame (310, 310') of the carrier (300) are concave or convex in shape, and the second guiding means (400) of the infusion pump (120) are convex or concave in shape.

## Patentansprüche

1. Träger (300) zum Halten einer Infusionspumpe (120) sodass die Infusionspumpe an dem Körper eines Benutzers getragen werden kann, wobei der Träger umfasst:
einen Trägerrahmen (310, 310');
ein Klebemittel (350, 350') sodass der Träger auf der Haut eines Benutzers befestigt werden kann;
eine erste Führungseinrichtung (340, 341) an dem Trägerrahmen, die mit einer komplementären zweiten Führungseinrichtung (400), die an der Infusionspumpe vorgesehen ist, ineinandergreifen kann, um die Infusionspumpe an dem Träger anzubringen und die Infusionspumpe an ihrem Platz auf dem Trägerrahmen zu halten;
**dadurch gekennzeichnet, dass**
das Klebemittel einen Befestigungsbereich (320) umfasst, wobei der Befestigungsbereich entweder auf einen zentralen Punkt oder eine Linie entlang einer horizontalen Achse (X-X) des Klebemittels beschränkt ist;
und der Trägerrahmen einen oder mehrere Befestigungsbereiche (330, 330', 331) aufweist, woder Trägerrahmen an dem Befestigungsbereich des Klebemittels befestigt ist.

2. Träger nach Anspruch 1, wobei der Trägerrahmen (310) zwei auf einer Linie angeordnete Befestigungsbereiche (330', 331) umfasst.

3. Träger nach einem der vorhergehenden Ansprüche, wobei der Trägerrahmen (310, 310') mindestens eine Austrennung (320) derart aufweist, dass das Klebemittel (350) für einen Benutzer zugänglich ist.

4. Träger nach einem der vorhergehenden Ansprüche, wobei der Trägerrahmen (310, 310') ferner mindestens ein Befestigungsmittel (410) zum Befestigen (420) einer Infusionspumpe (120) an dem Träger (300) aufweist.

5. Träger nach Anspruch 4, wobei das mindestens eine Befestigungsmittel (410) einen Schnappverschluss umfasst.

6. Träger nach einem der vorhergehenden Ansprüche, wobei der Trägerrahmen (310, 310') ferner eine integrierte Nadel (500) zur Abgabe eines Fluids in einen Körper umfasst.

7. Träger nach einem der vorhergehenden Ansprüche, wobei der Träger (300) eine integrierte Nadel (500) zur Abgabe eines Fluids in einen Körper aufweist, mit einer Kanüle (510), die in das Gewebe des Körpers einzuführen ist, z. B. eine Stahlkanüle oder eine flexible Kanüle, und ein Septum (520), das die Kanüle von außen derart hermetisch abdichtet, dass, wenn eine Infusionspumpe (120) mit dem Träger verbunden ist, eine Verbindungsnadel der Infusionspumpe, die mit einem Fluidkanal (140) der Infusionspumpe verbunden ist, das Septum (520) durchdringen und eine Fluidverbindung zwischen dem Fluidkanal (140) der Infusionspumpe (120) und der Kanüle (510) herstellen kann.

8. Träger nach einem der vorhergehenden Ansprüche, wobei der Trägerrahmen (310, 310') mit dem Befestigungsbereich (330, 330') des Trägerrahmens durch eine Verbindungsschiene (332) verbunden ist.

9. Infusionssystem (100), umfassend eine Infusionspumpe (120) und einen Träger (300) nach einem der vorhergehenden Ansprüche, wobei die Infusionspumpe eine zweite Führungseinrichtung (400) umfasst, die mit einer ersten Führungseinrichtung (340, 341) eines Trägerrahmens (310, 310') des Trägers ineinandergreifen kann, und dass die Infusionspumpe an dem Träger befestigt und auf dem Trägerrahmen durch Ineinandergreifen der ersten Führungseinrichtung und der zweiten Führungseinrichtung festgehalten werden kann.

10. Infusionssystem nach Anspruch 9, wobei die erste Führungseinrichtung des Trägerrahmens (310, 310') des Trägers (300) konkav oder konvex ausgebildet ist und die zweite Führungseinrichtung (400) der Infusionspumpe (120) konvex oder konkav ausgebildet ist.

## Revendications

1. Support (300) pour le maintien d'une pompe à perfusion (120) de manière à ce que la pompe à perfusion puisse être portée sur le corps d'un utilisateur, ce support comprenant :
un cadre de support (310,310');
un moyen adhésif (350,350') tel que le support puisse être fixé à la peau d'un utilisateur;
un premier moyen de guidage (340,341) sur le cadre de support pouvant s'ajuster avec un second moyen de guidage supplémentaire (400) prévu sur la pompe à perfusion afin de fixer la pompe à perfusion au support et de maintenir la pompe à perfusion en place sur le cadre de support;
**caractérisé en ce que**
le moyen adhésif comprend une zone de fixation (320), la zone de fixation étant restreinte soit à un point central, soit à une ligne le long d'un axe horizontal (X-X) du moyen adhésif; et le cadre de support comportant une ou plusieurs zones de fixation (330, 330', 331) où le cadre de support est fixé à la zone de fixation du moyen adhésif.

2. Support selon la revendication 1, dans lequel le cadre de support (310) comprend deux zones de fixation (330', 331) disposées sur une ligne.

3. Support selon l'une quelconque des revendications précédentes, dans lequel le cadre de support (310) comprend au moins une découpe (320) de manière à ce que le moyen adhésif (350) soit accessible à un utilisateur.

4. Support selon l'une quelconque des revendications précédentes, dans lequel le cadre de support (310, 310') comprend en outre au moins un moyen de fixation (410) pour fixer (420) une pompe à perfusion (120) au support (300).

5. Support selon la revendication 4, dans lequel l'au moins un moyen de fixation (410) comprend une attache à déclic.

6. Support selon l'une quelconque des revendications précédentes, dans lequel le cadre de support (310,310') comprend en outre une aiguille intégrée (500) pour la délivrance d'un fluide dans un corps.

7. Support selon l'une quelconque des revendications précédentes, dans lequel le support (300) comprend une aiguille intégrée (500) pour la délivrance d'un fluide dans un corps, comportant une canule (510) à insérer dans le tissu du corps, par exemple une canule en acier ou une canule souple, et un septum (520) fermant hermétiquement la canule par rapport à l'extérieur de sorte que, lorsqu'une pompe à perfusion (120) est connectée au support, une aiguille de connexion de la pompe à perfusion qui est connectée au canal à fluide (140) de la pompe à perfusion peut pénétrer dans le septum (520) et établir une connexion fluidique entre le canal à fluide (140) de la pompe à perfusion (120) et la canule (510).

8. Support selon l'une quelconque des revendications précédentes, dans lequel le cadre de support (310,310') est connecté à la zone de fixation (330,330') du cadre de support par une barre de connexion (332).

9. Système de perfusion (100) comprenant une pompe à perfusion (120), et un support (300) selon l'une quelconque des revendications précédentes, la pompe à perfusion comprenant un second moyen de guidage (400) qui peut s'ajuster avec le premier moyen de guidage (340,341) d'un cadre de support (310,310') du support, et que la pompe à perfusion peut être fixée au support et rester en place sur le cadre de support en s'ajustant avec le premier moyen de guidage et le second moyen de guidage.

10. Système de perfusion selon la revendication 9, dans lequel le premier moyen de guidage (340,341) du cadre de support (310,310') du support (300) a une forme concave ou convexe, et le second moyen de guidage (400) de la pompe à fusion (120) a une forme convexe ou concave.
